(19) Europäisches Patentamt
European Patent Office
Office européen des brevets

(11) EP 1 112 367 B1

(12) **EUROPÄISCHE PATENTSCHRIFT**

(45) Veröffentlichungstag und Bekanntmachung des Hinweises auf die Patenterteilung:
**29.12.2004 Patentblatt 2004/53**

(21) Anmeldenummer: **99946125.4**

(22) Anmeldetag: **04.09.1999**

(51) Int Cl.[7]: **C12N 15/52**, C12N 15/11, C12N 15/82, C12N 9/00, C12N 5/10, C07K 16/40, A01H 5/00, A01H 5/10

(86) Internationale Anmeldenummer:
**PCT/EP1999/006522**

(87) Internationale Veröffentlichungsnummer:
**WO 2000/014247 (16.03.2000 Gazette 2000/11)**

(54) **MITTEL UND VERFAHREN ZUR VERÄNDERUNG DES GLUTAMINSTOFFWECHSELS IN PFANZEN, INSBESONDERE IN ZUCKERRÜBEN**

AGENTS AND METHODS FOR MODIFYING THE GLUTAMINE METABOLISM IN PLANTS, ESPECIALLY IN SUGAR BEET

AGENTS ET PROCEDE POUR LA MODIFICATION DU METABOLISME GLUTAMIQUE DANS DES PLANTES, EN PARTICULIER DANS DES BETTERAVES A SUCRE

(84) Benannte Vertragsstaaten:
**AT BE CH CY DE DK ES FI FR GB GR IE IT LI LU MC NL PT SE**

(30) Priorität: **08.09.1998 DE 19840964**

(43) Veröffentlichungstag der Anmeldung:
**04.07.2001 Patentblatt 2001/27**

(73) Patentinhaber: **SÜDZUCKER AKTIENGESELLSCHAFT MANNHEIM/OCHSENFURT**
**68165 Mannheim (DE)**

(72) Erfinder:
- **TISCHNER, Rudolf**
**D-37077 Göttingen (DE)**
- **HOFFMANN, Guido**
**D-37083 Göttingen (DE)**

(74) Vertreter: **Schrell, Andreas, Dr. et al**
**Gleiss & Grosse**
**Leitzstrasse 45**
**70469 Stuttgart (DE)**

(56) Entgegenhaltungen:
**WO-A-91/13159** **WO-A-95/09911**
**WO-A-97/38115**

- **TEMPLE SJ. ET AL. : "Modulation of glutamine synthetase gene expression in tobacco by the production of an alfalfa glutamine synthetase gene in sense and tisense orientation: molecular and biochemical analysis." MOL GEN GENET 1993 JAN;236(2-3):315-25, XP002016640**
- **TEMPLE SJ. ET AL. : "Down-regulation of specific members of the glutamine synthetase gene family in alfalfa by antisense RNA technology." PLANT MOL BIOL 1998 JUN;37(3):535-47, XP002130886 & TEMPLE, S.J., ET AL. : "characterization of a nodule-enhanced glutamine synthetase from alfalfa: nucleotide sequence, in-situ localisation, and transcript analysis" EMBL SEQUENCE DATA LIBRARY, 5. November 1994 (1994-11-05), heidelberg, germany**
- **MAECK G ET AL: "---GLUTAMINE--- SYNTHETASE OLIGOMERS AND ISOFORMS IN SUGAR BEET BETA-VULGARIS L." PLANTA (HEIDELB), (1990) 181 (1), 10-17. , XP000879497**
- **VINCENT R. ET AL. : "Overexpression of a soybean gene encoding cytosolic glutamine synthetase shoots of transgenic Lotus corniculatus L. plants triggers changes in monium assimilation and plant development." PLANTA 1997;201(4):424-33, XP000879495**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

(Forts. nächste Seite)

- **MAECK, GISELA: "Glutamine synthetase isoenzymes, oligomers, and subunits from hairy roots of ---Beta--- ---vulgaris--- var. lutea" PLANTA (1998), 205(1), 113-120 , Mai 1998 (1998-05), XP000879496**
- **BRECHLIN P ET AL: "Changes in the isoform pattern and subunit composition of GS-1 in sugar beet leaves dependent on leaf age" JOURNAL OF PLANT PHYSIOLOGY, (OCT 1999) VOL. 155, NO. 4-5, PP. 497-502. PUBLISHER: GUSTAV FISCHER VERLAG, VILLENGANG 2, D-07745 JENA, GERMANY. , XP000879494**


Bemerkungen:

Die Akte enthält technische Angaben, die nach dem Eingang der Anmeldung eingereicht wurden und die nicht in dieser Patentschrift enthalten sind.

SEQUENZPROTOKOLL

(1) ALLGEMEINE ANGABEN:

(i) ANMELDER:

(A) NAME: Suedzucker Aktiengesellschaft Mannheim/Ochsenfurt
(B) STRASSE: Maximilianstrasse 10
(C) ORT: MANNHEIM
(E) LAND: BRD
(F) POSTLEITZAHL: 68165

(ii) BEZEICHNUNG DER ERFINDUNG: Genetisch modifizierte Zuckerruebe

(iii) ANZAHL DER SEQUENZEN: 3

(iv) COMPUTER-LESBARE FASSUNG:

(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.30 (EPA)

(2) ANGABEN ZU SEQ ID NO: 1:

(i) SEQUENZKENNZEICHEN:

(A) LÄNGE: 1068 Basenpaare
(B) ART: Nucleotid
(C) STRANGFORM: Einzelstrang
(D) TOPOLOGIE: linear

(ii) ART DES MOLEKÜLS: cDNA

(vi) URSPRÜNLICHE HERKUNFT:

(A) ORGANISMUS: Beta vulgaris

(xi) SEQUENZBESCHREIBUNG: SEQ ID NO: 1 :

```
ATGGCTCTTC TTAACGATCT AATTAACCTT AATCTCTCAG AAACATCTGA CAAGATTATT     60
GCTGAGTATA TATGGATCGG AGGATCTGGG TTGGATATGA GAAGCAAAGC AAGGACATTA    120
ACAGGGCCAA TAAGTGATCC TGCAAAATTA CCAAAATGGA ATTACGATGG ATCAAGTACT    180
AATCAAGCTC CTGGTGAAGA TAGTGAAGTC ATTTTATACC CTCAGGCTAT TTTCAAGGAT    240
CCATTCAGAA GAGGAGACAA TATCCTAGTT ATGTGTGATG CATACACCCC AGCTGGAGAA    300
CCAATTCCAA CAAACAAGAG GTACAATGCT GAGAAAATCT TCAGCCACCC AGACGTTGTC    360
GCTGAGGAGC CATGGTATGG AATTGAACAA GAATACACAC TTCTTCAGAA GGATATCAAC    420
TGGCCTCTTG GATGGCCAAC TGGTGGCTTC CCTGGTCCTC AGGGACCATA CTATTGTGGT    480
GTAGGGGCTG ATAAATCTTT CGGGCGAGAC ATTGTAGATG CTCACTATAA GGCTTGCATC    540
TATGCTGGTG TCAATATCAG TGGAATTAAT GGTGAAGTCA TGCCAGGACA GTGGGAATTC    600
CAAGTTGGTC CTACTGTTGG AATTTCATCT GGTGATCAAG TCTGGGTTGC TAGATACATT    660
CTTGAGAGAA TTGCTGAGAT TGCCGGAGTT GTTGTGTCTT TTGACCCCAA ACCAGTGAAG    720
GGTGATTGGA ATGGTGCTGG TGCTCACACC AACTACAGCA CTAAGTCGAT GAGGGAAGAT    780
GGTGGGATCA ATGTTATAAA GGCTGCCATC GAGAAGTTGA GTCTCCGTCA CAAGGAGCAC    840
ATTGCTGCCT ACGGGGAGGG CAACGAGAGG AGGCTCACTG GTCGTCATGA GACAGCCGAT    900
ATCACCACTT TCTCCTGGGG TGTTGCAAAT AGGGGTGCCT CTGTTCGTGT TGGCCGTGAC    960
ACAGAGAAAG ATGGCAAAGG TTACTTTGAA GACAGAAGGC CAGCATCAAA CATGGATCCA   1020
TATGTGGTTA CCTCCATGAT TGCTGAAACA ACTATCCTCG GAAAGCCT               1068
```

(2) ANGABEN ZU SEQ ID NO: 2:

(i) SEQUENZKENNZEICHEN:

(A) LÄNGE: 356 Aminosäuren
(B) ART: Aminosäure
(C) STRANGFORM: Einzelstrang
(D) TOPOLOGIE: linear

(ii) ART DES MOLEKÜLS: Protein

(vi) URSPRÜNLICHE HERKUNFT:

(A) ORGANISMUS: Beta vulgaris

(xi) SEQUENZBESCHREIBUNG: SEQ ID NO: 2:

```
Met Ala Leu Leu Asn Asp Leu Ile Asn Leu Asn Leu Ser Glu Thr Ser
1               5                   10                  15

Asp Lys Ile Ile Ala Glu Tyr Ile Trp Ile Gly Gly Ser Gly Leu Asp
            20                  25                  30

Met Arg Ser Lys Ala Arg Thr Leu Thr Gly Pro Ile Ser Asp Pro Ala
        35                  40                  45

Lys Leu Pro Lys Trp Asn Tyr Asp Gly Ser Ser Thr Asn Gln Ala Pro
    50                  55                  60

Gly Glu Asp Ser Glu Val Ile Leu Tyr Pro Gln Ala Ile Phe Lys Asp
65                  70                  75                  80

Pro Phe Arg Arg Gly Asp Asn Ile Leu Val Met Cys Asp Ala Tyr Thr
                85                  90                  95

Pro Ala Gly Glu Pro Ile Pro Thr Asn Lys Arg Tyr Asn Ala Glu Lys
            100                 105                 110

Ile Phe Ser His Pro Asp Val Val Ala Glu Glu Pro Trp Tyr Gly Ile
        115                 120                 125

Glu Gln Glu Tyr Thr Leu Leu Gln Lys Asp Ile Asn Trp Pro Leu Gly
    130                 135                 140

Trp Pro Thr Gly Gly Phe Pro Gly Pro Gln Gly Pro Tyr Tyr Cys Gly
145                 150                 155                 160

Val Gly Ala Asp Lys Ser Phe Gly Arg Asp Ile Val Asp Ala His Tyr
                165                 170                 175

Lys Ala Cys Ile Tyr Ala Gly Val Asn Ile Ser Gly Ile Asn Gly Glu
            180                 185                 190
```

```
Val Met Pro Gly Gln Trp Glu Phe Gln Val Gly Pro Thr Val Gly Ile
        195                 200                     205

Ser Ser Gly Asp Gln Val Trp Val Ala Arg Tyr Ile Leu Glu Arg Ile
    210                 215                 220

Ala Glu Ile Ala Gly Val Val Val Ser Phe Asp Pro Lys Pro Val Lys
225                 230                 235                 240

Gly Asp Trp Asn Gly Ala Gly Ala His Thr Asn Tyr Ser Thr Lys Ser
                245                 250                 255

Met Arg Glu Asp Gly Gly Ile Asn Val Ile Lys Ala Ala Ile Glu Lys
            260                 265                 270

Leu Ser Leu Arg His Lys Glu His Ile Ala Ala Tyr Gly Glu Gly Asn
        275                 280                 285

Glu Arg Arg Leu Thr Gly Arg His Glu Thr Ala Asp Ile Thr Thr Phe
    290                 295                 300

Ser Trp Gly Val Ala Asn Arg Gly Ala Ser Val Arg Val Gly Arg Asp
305                 310                 315                 320

Thr Glu Lys Asp Gly Lys Gly Tyr Phe Glu Asp Arg Arg Pro Ala Ser
                325                 330                 335

Asn Met Asp Pro Tyr Val Val Thr Ser Met Ile Ala Glu Thr Thr Ile
            340                 345                 350

Leu Gly Lys Pro
        355
```

(2) ANGABEN ZU SEQ ID NO: 3:

(i) SEQUENZKENNZEICHEN:

(A) LÄNGE: 1534 Basenpaare
(B) ART: Nucleotid
(C) STRANGFORM: Einzelstrang
(D) TOPOLOGIE: linear

(ii) ART DES MOLEKÜLS: cDNA

(vi) URSPRÜNLICHE HERKUNFT:

(A) ORGANISMUS: Beta vulgaris

(xi) SEQUENZBESCHREIBUNG: SEQ ID NO: 3:

GAATTGGGTA CCGGGCCCCC CCTCGAGGTC GACGGTATCG ATAAGCTTGA TATCGAATTC 60

CGTTGCTGTC GCCGTTGCTG TCGCCGTTGC TGTCGCCGTT GCTGTCGCCG TTGCTGTCGG 120

CGGCCGCTTT TTTTTTTTTT TTTTTCTCTC TCTTCATTTT CTTCACTTTT TCTTCTTCAT 180

AACAAAAAAT CATCAATCAT GGCTCTTCTT AACGATCTAA TTAACCTTAA TCTCTCAGAA 240

ACATCTGACA AGATTATTGC TGAGTATATA TGGATCGGAG GATCTGGGTT GGATATGAGA 300

AGCAAAGCAA GGACATTAAC AGGGCCAATA AGTGATCCTG CAAAATTACC AAAATGGAAT 360

TACGATGGAT CAAGTACTAA TCAAGCTCCT GGTGAAGATA GTGAAGTCAT TTTATACCCT 420

CAGGCTATTT TCAAGGATCC ATTCAGAAGA GGAGACAATA TCCTAGTTAT GTGTGATGCA 480

TACACCCCAG CTGGAGAACC AATTCCAACA AACAAGAGGT ACAATGCTGA GAAAATCTTC 540

AGCCACCCAG ACGTTGTCGC TGAGGAGCCA TGGTATGGAA TTGAACAAGA ATACACACTT 600

CTTCAGAAGG ATATCAACTG GCCTCTTGGA TGGCCAACTG GTGGCTTCCC TGGTCCTCAG 660

GGACCATACT ATTGTGGTGT AGGGGCTGAT AAATCTTTCG GGCGAGACAT TGTAGATGCT 720

CACTATAAGG CTTGCATCTA TGCTGGTGTC AATATCAGTG GAATTAATGG TGAAGTCATG 780

CCAGGACAGT GGGAATTCCA AGTTGGTCCT ACTGTTGGAA TTTCATCTGG TGATCAAGTC 840

TGGGTTGCTA GATACATTCT TGAGAGAATT GCTGAGATTG CCGGAGTTGT TGTGTCTTTT 900

GACCCCAAAC CAGTGAAGGG TGATTGGAAT GGTGCTGGTG CTCACACCAA CTACAGCACT 960

AAGTCGATGA GGGAAGATGG TGGGATCAAT GTTATAAAGG CTGCCATCGA GAAGTTGAGT 1020

```
CTCCGTCACA AGGAGCACAT TGCTGCCTAC GGGGAGGGCA ACGAGAGGAG GCTCACTGGT    1080
CGTCATGAGA CAGCCGATAT CACCACTTTC TCCTGGGGTG TTGCAAATAG GGGTGCCTCT    1140
GTTCGTGTTG GCCGTGACAC AGAGAAAGAT GGCAAAGGTT ACTTTGAAGA CAGAAGGCCA    1200
GCATCAAACA TGGATCCATA TGTGGTTACC TCCATGATTG CTGAAACAAC TATCCTCGGA    1260
AAGCCTTGAA AACATTATAA TTAATTCCGC ATTTCAATAT ATCTTCATGT GAATTTGGAT    1320
AACAATTGGA GTTGTATTTG TGATTAGACA ACTATTTTAC ATTTTCTCCA GGTTTGAAAT    1380
AAATTATCTG TACGTTGTTC TGAATTTTTT TCCTTATCTT TTGCTTGATA GTACAAGGAA    1440
ATTTGGCTTC ACATGTTAAT ATGATGGCTT CTTTGGTAAA AAAAAAAAAA AAAAAAAAAA    1500
AAAAAAAAGC GGCCGCCACC GCGGTGGAGC TCCA                                1534
```

## Beschreibung

Die vorliegende Erfindung betrifft die Nucleotidsequenz einer Glutaminsynthetase aus der Zuckerrübe, einen Vektor enthaltend diese Nucleotidsequenz, Zellen, die mit diesem Vektor transformiert sind, von dieser Nucleotidsequenz codierte Proteine, Pflanzen, die mit dieser Nucleotidsequenz transformiert wurden sowie Verfahren zur genetischen Modifikation von Pflanzen, insbesondere Zuckerrüben.

Die Akkumulation von Glutamin als die hauptsächlich in der Speicherrübe, also dem Speicherorgan der Zuckerrübe, vorkommende α-Amino-N-Komponente, die auch als schädlicher Stickstoff bezeichnet wird, bringt erhebliche Probleme in der Zuckerproduktion mit sich. Die im allgemeinen durch Alkalisierung durchgeführte Kompensation der durch diese Komponente verursachten Ansäuerung des Rübensaftes ist kostenintensiv, führt zu schnellerem Verschleiß der Produktionsanlagen und bringt schließlich auch erhebliche Umweltbelastungen mit sich, die ebenfalls nur unter Einsatz kostenintensiver Maßnahmen verhindert werden können. Die Synthese dieses Glutamins erfolgt in der Pflanze durch eine Amidierung der Glutaminsäure, indem $NH_4^+$ unter ATP-Verbrauch am C-4 des Glutamats gebunden wird. Dieser Schritt wird durch das Enzym Glutaminsynthetase (im folgenden kurz GS genannt) katalysiert. Dieses Enzym liegt sowohl im Chloroplasten als auch im Cytoplasma der Pflanzenzelle vor. Im Chloroplasten kommt das Enzym als von einem Gen codiertes Tetramer aus bis zu fünf Untereinheiten vor (GS-2). Im Cytoplasma kommt das Enzym nach bisherigen Erkenntnissen üblicherweise als von mehreren Genen codiertes Heterooligomer (GS-1) vor. Die unterschiedlichen bekannten GS-1 Isoenzyme sind in der Regel Heterooktamere. Sechs verschiedene Isoformen der GS-1 wurden bisher aufgefunden. Die im Chloroplasten lokalisierte Glutaminsynthetase, also die GS-2, hat vorwiegend die Funktion, das während der Photorespiration anfallende $NH_4^+$ zu binden und das aus der Nitratreduktion stammende $NH_4^+$ in organische Verbindungen zu überführen. Die Funktion der GS-1 liegt dagegen überwiegend in katabolischen Abbauwegen, in deren Verlauf das aus der Proteindegradation anfallende $NH_4^+$ fixiert wird. Derartige Abbauwege spielen insbesondere während des Alterns des Blattes, das heißt während der Seneszenz, eine Rolle, wobei das entstehende Glutamin aus dem Blatt in Speicherorgane exportiert wird.

Im Gegensatz zur GS-2 im Chloroplasten ist über die GS-1 im Cytoplasma relativ wenig bekannt. Dies betrifft vor allem ihre Funktion in der Zelle aber auch ihre Regulation. Im Gegensatz zur GS-2 ging man bisher davon aus, daß die GS-1 von mehreren Genen codiert wird, wobei deren Anzahl variabel ist. Die Gene zeigen untereinander Homologien, sind jedoch eindeutig voneinander zu trennen (Brears et al., Plant J. 1 (1991), 235 bis 244; Edwards et al., Plant Cell 1 (1989), 241 bis 248). Zusätzlich scheinen die GS-1 Gene, die jeweils eine Untereinheit des oktameren Holoenzyms codieren, unterschiedlich reguliert zu sein (Petermann and Goodman MGG 230 (1991), 145 bis 154). Dies erschwert eine kontrollierte externe Beeinflussung des Glutaminstoffwechsels über eine Modifikation der GS-1.

Auch die Lokalisation der GS-1 in der Pflanze ist noch weitgehend unaufgeklärt. Aus Edwards et al. (a.a.O.) ist bekannt, daß eine Isoform ausschließlich im Phloem exprimiert werden soll, wo sie möglicherweise eine Rolle im interzellulären Transport spielt. Sakurai et al. (Planta 200 (1996), 306 bis 311) berichtet, daß in Reis eine Isoform der GS-1 an den Leitbündeln vorliegt und offensichtlich eine Rolle im Export von Glutamin aus Blättern spielt. Bekannt ist es auch, daß Tabak- und Alfalfapflanzen, die mit einem GS-1 Gen aus Lotus corniculatus transformiert wurden, Expression in den Blüten zeigen (Carrayol et al., Plant Sci 125 (1997), 75 bis 85). Bekannt ist es ferner, daß die Zusammensetzung und die Lokalisation von GS-1 Holoenzymen in den Wurzelknöllchen von Lotus corniculatus stark variieren können. Maeck et al. (Planta 181 (1990), 10 bis 17) offenbart verschiedene GS-Isoformen in Zuckerrübe, und zwar in unterschiedlichen Entwicklungsstadien und Organen.

Eine gezielte, mit molekularbiologischen Methoden durchgeführte Reduzierung des Glutamingehaltes in Pflanzen, insbesondere in Speicherorganen von Pflanzen, ist bisher nicht bekannt. Wesentliche Schwierigkeiten, die bei der gezielten Reduzierung der Glutaminsynthetaseaktivität in Pflanzen und der letztendlich erwünschten Reduzierung des Stickstoffgehaltes in Speicherorganen von Pflanzen auftreten, liegen darin, daß die Glutaminsynthetaseaktivität gewebe- und zeitspezifisch so eingeschränkt werden muß, daß in dem Zielorgan, beispielsweise der Speicherrübe einer Zuckerrübe, der Stickstoffgehalt reduziert wird. Dabei darf die Zuckerrübe keinerlei Beeinträchtigungen ihrer sonstigen Funktionen und Eigenschaften erfahren. Es muß vielmehr sichergestellt sein, daß die gesamte Physiologie der Zuckerrübe intakt bleibt und nur spezifisch der Stickstoffgehalt im Speicherorgan der Zuckerrübe reduziert wird. Aufgrund der geschilderten Probleme hinsichtlich der Lokalisation der GS-1 und Unklarheiten bezüglich der Zeitspezifität ihrer Aktivität sind keinerlei erfolgreiche Experimente bekannt geworden, gemäß derer der Stickstoffgehalt in Speicherorganen von Zuckerrüben über eine Modulation der GS-1 Aktivität reduziert werden konnte.

Das der vorliegenden Erfindung zugrundeliegende technische Problem besteht also darin, Mittel und Verfahren zur Verfügung zu stellen, die gezielt, das heißt gewebe- und zeitspezifisch, eine Verringerung des Stickstoffgehaltes in dem Speicherorgan einer Pflanze, insbesondere einer Zuckerrübe, ermöglichen, ohne dabei die Lebens-, Wachstums- und Fortpflanzungsfunktionen der Zuckerrübe und ihren wirtschaftlichen Wert zu beeinträchtigen.

Die vorliegende Erfindung löst dieses Problem durch die Bereitstellung einer isolierten und gereinigten Nucleotidsequenz gemäß Hauptanspruch und diese Nucleotidsequenz enthaltender Vektoren, die für eine Untereinheit der GS-1 der Zuckerrübe codieren. Die vorliegende Erfindung löst das technische Problem auch durch die Bereitstellung des isolierten und gereinigten von dieser Nucleotidsequenz codierten Proteins, insbesondere der Aminosäuresequenz der GS-1 Untereinheit aus Zuckerrübe sowie durch Verfahren zur genetischen Modifikation von Pflanzen, nämlich Zuckerrüben, wobei der Gehalt an Glutaminsynthetase in der Pflanze, insbesondere in deren seneszenten Blättern, geändert wird, insbesondere verringert wird, indem Zellen dieser Pflanze mit den genannten Vektoren transformiert und aus den transformierten Zellen intakte, fortpflanzungsfähige stabil transformierte transgene Pflanzen regeneriert werden, in deren seneszenten Blättern die Aktivität der GS-1 reduziert oder vollständig unterdrückt ist.

Die Erfindung ist insbesondere deshalb überraschend und vorteilhaft, als daß die durch die erfindungsgemäße Nucleotidsequenzen codierte Untereinheit die einzige Untereinheit der in den seneszenten Blättern der Zuckerrübe vorkommenden Isoform der oligomeren GS-1 darstellt. Demgemäß stellt die Erfindung auch die überraschende Erkenntnis bereit, daß diese Isoform der GS-1 ein Homooktamer ist. Dies ermöglicht in überraschender Weise die Aktivität dieses Enzyms durch die Beeinflussung der Expression eines einzigen Gens, nämlich des die GS-1 Untereinheit codierenden Gens, zu beeinflussen, insbesondere deren Expression zu verhindern oder zu reduzieren. Die Erfindung ist auch insofern überraschend, als daß die erfindungsgemäß bereitgestellte homooligomere Isoform der GS-1 nur im Stadium der Seneszenz auftritt und demgemäß neben der bereits erwähnten Gewebespezifität hinsichtlich der Lokalisation im Blatt auch eine Zeitspezifität hinsichtlich des Auftretens während der Seneszenz aufweist. Die vorliegende Erfindung stellt überraschenderweise also Mittel und Verfahren zur Verfügung, das qualitative und/oder quantitative Auftreten einer nur in seneszenten Blättern der Zuckerrübe auffindbaren homooktameren GS-1 Isoform zu beeinflussen. Die erfindungsgemäßen Nucleotidsequenzen können auch Verwendung bei der Clonierung homologer Gene, insbesondere derer codierender Regionen, in anderen Geweben oder sogar anderen Pflanzen und Organismen finden. Insbesondere können bei Verwendung der vorliegenden Nucleotidsequenz als Hybridisierungssonde in homologen oder heterologen Systemen auch mit dieser Sequenz assoziierte endogene regulatorische nicht-codierende Nucleotidsequenzen identifiziert und isoliert werden, die beispielsweise zeit- und gewebespezifische Expression vermitteln.

Die Erfindung löst das vorliegende technische Problem insbesondere durch die Bereitstellung einer aus Zuckerrübe (Beta vulgaris) stammenden Nucleotidsequenz für die Modulation der Expression, insbesondere für die Unterdrückung der Expression, eines Proteins mit der Aktivität einer Glutaminsynthetase, insbesondere der Aktivität einer GS-1, die ausgewählt ist aus der Gruppe bestehend aus

a) der DNA-Sequenz der SEQ ID Nr. 1 oder 3,
b) einer Nucleotidsequenz, die die Aminosäuresequenz der SEQ ID Nr. 2 codiert,
c) einer zu den Nucleotidsequenzen von a) oder b) komplementären Nucleotidsequenz, und
d) einer zu den Nucleotidsequenzen von a) mindestens 80 % Homologie aufweisenden Nucleotidsequenz.

Die erfindungsgemäße Nucleotidsequenz ist funktional dadurch gekennzeichnet, daß sie in einer mit ihr transformierten, eine endogene GS-1 codierende Sequenz aufweisenden Zelle die Aktivität der GS-1 Aktivität dieser transformierten Zelle moduliert, zum Beispiel die GS-1 Aktivität erhöht, zum Beispiel durch Überexpression, oder die GS-1 Aktivität reduziert oder vollständig unterdrückt, zum Beispiel dadurch, daß die erfindungsgemäße Nucleotidsequenz in Form eines antisense-Konstrukts transformiert wird, welches die endogene GS-1 Translation inhibiert.

Die erfindungsgemäße Nucleotidsequenz kann eine DNA-Sequenz, zum Beispiel eine genomische, gegebenenfalls durch Introns unterbrochene DNA-Sequenz oder cDNA-Sequenz sein, sie kann aber auch eine RNA-Sequenz, zum Beispiel eine mRNA-Sequenz oder synthetisch hergestellt sein. Die vorliegende Erfindung betrifft sowohl die Sense- als auch die Antisensenucleotidsequenzen. Die erfindungsgemäßen Nucleotidsequenzen können sogenannte full length-Sequenzen sein, also Sequenzen, die ein vollständiges Protein mit der Aktivität einer Glutaminsynthetase, insbesondere der GS-1, der Zuckerrübe codieren, gegebenenfalls einschließlich der Translationsinitiationsstelle. Die Erfindung betrifft jedoch auch Teilsequenzen solcher Nucleotidsequenzen, insbesondere solche, die der Modulation der Expression des Proteins mit der Aktivität einer Glutaminsynthetase, insbesondere einer GS-1 aus Zuckerrübe, dienen. Demgemäß kann die erfindungsgemäße Nucleotidsequenz auch in Transcriptions- oder Translationseinheit mit weiteren Nucleotidsequenzen ein Fussionsgen bilden.

Im Zusammenhang mit der vorliegenden Erfindung wird unter einer Hybridisierung eine Vorhybridisierung, eine Hybridisierung und ein anschließendes Waschen verstanden. Die Vorhybridisierung findet vorzugsweise in einer wässrigen Lösung aus 6 x SSPE, 0,1% SDS und 5 x Denhardt's Reagenz sowie 500 µg/ml denaturiertem Heringssperma für 3 Stunden bei 60°C, besonders vorzugsweise bei 65°C, statt. Die Hybridisierung findet vorzugsweise in einer wässrigen Lösung aus 3 x SSPE, 0,1% SDS, 5 x Denhardt's Reagenz und 500 µg/ml denaturiertem Heringssperma für 16 Stunden bei 60°C, besonders vorzugsweise bei 65°C, statt. Das Waschen findet vorzugsweise in einer wässrigen Lösung aus 2 x SSPE und 0,1% SDS bei Raumtemperatur für 10 Minuten statt, wobei sich daran in einer wässrigen Lösung aus 2 x SSPE und 0,1% SDS ein weiterer Waschschritt bei 60°C, besonders bevorzugt bei 65°C, für 15 Minuten und mit einer wässrigen Lösung aus 0,4 x SSPE und 0,02% SDS ein letzter Waschschritt bei 60°C, besonders bevorzugt bei 65°C, für 15 Minuten anschließt.

In besonders bevorzugter Ausführungsform wird eine Vorhybridisierung in einer wässrigen Lösung aus 6 x SSPE, 0,1% SDS und 5 x Denhardt's Reagenz sowie 500 µg/ml denaturiertem Heringssperma für 3 Stunden bei 65°C durchgeführt. Die Hybridisierung findet in einer wässrigen Lösung aus 3 x SSPE, 0,1% SDS, 5 x Denhardt's Reagenz und 500 µg/ml denaturiertem Heringssperma für 16 Stunden bei 68°C statt. Das Waschen findet in einer wässrigen Lösung aus 2 x SSPE und 0,1% SDS bei 68°C für 15 Minuten statt, wobei sich daran in einer wässrigen Lösung aus 1 x SSPE und 0,1% SDS ein weiterer Waschschritt bei 68°C für 15 Minuten und mit einer wässrigen Lösung aus 0,1 x SSPE und 0,1% SDS ein letzter Waschschritt bei 68°C für 15 Minuten anschließt.

Selbstverständlich betrifft die vorliegende Erfindung auch Modifikationen der vorgenannten Sequenzen, insbesondere solche, die gegenüber den in SEQ ID Nr. 1 oder 3 gezeigten Sequenzen Nucleotid-Additionen, -Deletionen, -Inversionen, -Substitutionen oder ähnliches aufweisen einschließlich chemischer Derivatisierungen oder Ersatz, Austausch oder Addition von ungewöhnlichen Nucleotiden.

Die Erfindung betrifft auch Nucleotidsequenzen, die einen Homologiegrad von mindestens 80 %, vorzugsweise 90 %, zu den in der SEQ ID Nr. 1 oder 3 gezeigten Sequenzen aufweisen.

Die Nucleotidsequenzen der vorliegenden Erfindung sind vorteilhaft insbesondere insofern, als daß sie der Modulation der Expression eines Proteins mit der Aktivität einer Glutaminsynthetase, insbesondere der GS-1 aus Zuckerrübe, dienen. Die erfindungsgemäßen Nucleotidsequenzen können eingesetzt werden, um die Menge der gebildeten Glutaminsynthetase, insbesondere der GS-1 aus Zuckerrübe, zu variieren, insbesondere zu reduzieren, in besonders bevorzugter Weise vollständig zu unterdrücken. In besonders bevorzugter Weise ermöglicht es die Erfindung, durch die Modulation der Expression in gewebe- und zeitspezifischer Weise die Einlagerung von Glutamin in das Speicherorgan der Zuckerrübe zu unterdrücken, ohne daß dabei zwangsläufig gewebe- und zeitspezifische Regulationselemente für das Transgen, also die erfindungsgemäße Nucleotidsequenz, eingesetzt werden müßten. Die erfindungsgemäßen Nucleotidsequenzen codieren nämlich die GS-1 der Zuckerrübe, die spezifisch nur in seneszenten Blättern auftritt, demgemäß eine Ortsspezifität im Hinblick auf die Expression in Blättern und eine Zeitspezifität im Hinblick auf die Expression während der Seneszenz aufweist. Die erfindungsgemäßen Nucleotidsequenzen ermöglichen es, diese spezifisch bei der Blattseneszenz auftretende Glutaminsynthetase mittels eines einzigen Genkonstrukts zu hemmen, da die GS-1 der Zuckerrübe ein Homooktamer ist und demgemäß mittels eines einzigen Genkonstrukts die Bildung sämtlicher Untereinheiten der GS-1 ausgeschaltet werden kann. Dementsprechend sieht die Erfindung in besonders bevorzugter Weise vor, Antisense-Konstrukte zu verwenden, die spezifisch die Bildung von Protein, also der GS-1 in den senseszenten Blättern der Zuckerrübe, unterbinden. Durch die Verwendung von Antisense-Konstrukten kann die spezifisch bei der Blattseneszenz auftretende GS-1 der Zuckerrübe in ihrer Expression inhibiert werden, so daß die Glutaminbildung und Einlagerung von Glutamin in die Speicherrübe verhindert wird. In vorteilhafter Weise wird das Wachstum der Zuckerrübe bei diesem Vorgang nicht beeinträchtigt, da die GS-2 durch die gentechnische Manipulation der Pflanzenzelle nicht betroffen wird.

Im Zusammenhang mit der vorliegenden Erfindung wird unter der Aktivität eines Proteins mit der Aktivität einer Glutaminsynthetase eine Aktivität verstanden, gemäß der enzymatisch $NH_4^+$ unter ATP-Einsatz am C-4 eines Glutamatmoleküls gebunden wird.

Im Zusammenhang mit der vorliegenden Erfindung wird unter der Modulation der Expression eines Proteins eine gezielte mit gentechnischen Verfahren erzielte Veränderung, das heißt Erhöhung oder Verringerung, der Proteinmenge in einer Zelle im Vergleich zur natürlicherweise in der betreffenden Zelle zur betreffenden Zeit vorhandenen Proteinmenge verstanden.

Die Modulation der Expression kann durch Beeinflussung der Translation oder Transcription der endogenen, das Protein codierenden Nucleotidsequenz geschehen, beispielsweise durch Einführen eines Antisense-Konstrukts, das die Menge an translatierbarer mRNA teilweise oder vollständig reduziert. Eine Erhöhung der Proteinmenge kann beispielsweise durch Einführen einer das Protein codierenden Nucleotidsequenz geschehen, die unter der Kontrolle überexprimierender Regulatorelemente steht oder durch Einführen vielfacher Genkopien.

Weitere alternative oder zusätzliche Modulationen können erzielt werden, indem gewebe- oder zeitspezifische, induzierbare oder konstitutiv exprimierende Regulatorelemente eingesetzt werden, die zu einem gegenüber dem natürlichen Expressionsmuster in der betreffenden Zelle und zu der betreffenden Zeit beziehungsweise zu dem betreffenden Entwicklungsstadium der Zelle oder der Pflanze geänderten Expressionsmuster führen.

Die vorliegende Erfindung betrifft in einer weiteren Ausführungsform Vektoren enthaltend mindestens eine der erfindungsgemäßen Nucleotidsequenzen. In besonders bevorzugter Ausführungsform der Erfindung ist ein derartiger Vektor als Plasmid oder viraler Vektor ausgeführt.

Die vorliegende Erfindung betrifft auch Vektoren der vorstehend genannten Art, wobei die mindestens eine Nucleotidsequenz der vorliegenden Erfindung unter der Kontrolle ebenfalls in dem Vektor vorhandener regulatorischer Nucleotidsequenzen steht, die beispielsweise 5', 3', 5' und 3' oder auch innerhalb der Nucleotidsequenz angeordnet sind. Diese regulatorischen Nucleotidsequenzen können zu der erfindungsgemäßen Nucleotidsequenz heterolog, das heißt aus einem anderen Organismus oder aus einem anderen Gen stammend, oder homolog sein, das heißt auch natürlicherweise mit den erfindungsgemäßen Nucleotidsequenzen in regulatorischer Einheit zusammen vorkommend sein.

Die Erfindung betrifft demgemäß auch Vektoren der vorstehend genannten Art, wobei 5'-wärts von der erfindungsgemäßen Nucleotidsequenz eine die Expression der erfindungsgemäßen Nucleotidsequenz steuernde Nucleotidsequenz, insbesondere ein Promotor, lokalisiert ist. In besonders bevorzugter Ausführungsform der Erfindung ist der Promotor der 35 S-Promotor des CaMV oder ein Promoter der T-DNA von Agrobacterium tumefaciens, beispielsweise der Promoter des Nopalin-Synthetase- oder Octopin-Synthetase-Gens.

Die Erfindung sieht in einer weiteren Ausführungsform vor, daß 3'-wärts von der erfindungsgemäßen Nucleotidsequenz eine Transkriptionsterminationseinheit, insbesondere ein 3'-Polyadenylierungssignal, lokalisiert ist, besonders bevorzugt das Polyadenylierungssignal des NOS-Gens von Agrobakterium tumefaciens.

Die vorliegende Erfindung sieht in einer weiteren bevorzugten Ausführungsform vor, daß die regulatorischen Sequenzen induzierbar sind, beispielsweise durch externe Faktoren.

Die Erfindung sieht in einer weiteren bevorzugten Ausführungsform vor, daß die regulatorischen Sequenzen der Expression der von diesen kontrollierten erfindungsgemäßen Nucleotidsequenzen Gewebeund/oder Zeitspezifität verleihen, beispielsweise eine Expression eines Antisense-Konstrukts spezifisch in Blättern während der Seneszenz bewirken.

Die Erfindung sieht auch vor, daß die erfindungsgemäßen Nucleotidsequenzen, gegebenenfalls in Einheit mit den ihnen zugeordneten regulatorischen Nucleotidsequenzen, zusammen mit Nucleotidsequenzen in dem Vektor angeordnet sind, die einen Transfer und eine Integration oder Rekombination der erfindungsgemäßen Nucleotidsequenzen gegebenenfalls mit den ihnen zugeordneten regulatorischen Nucleotidsequenzen in das Genom einer transformierten Zelle unterstützen. Die erfindungsgemäßen Nucleotidsequenzen können daher beispielsweise zwischen der linken und der rechten Borderregion, flankiert nur von jeweils einer Borderregion und/oder unterbrochen durch eine oder mehrere Borderregionen von Agrobacterium tumefaciens oder Agrobacterium rhizogenes angeordnet sein.

Die vorliegende Erfindung betrifft auch Zellen, enthaltend mindestens einen der vorgenannten Vektoren. In besonders bevorzugter Weise sind derartige Zellen Bakterienzellen, Hefezellen oder Pflanzenzellen, insbesondere Pflanzenzellen von monocotyledonen oder dicotyledonen Pflanzen, insbesondere Zuckerrüben. Die vorliegende Erfindung betrifft daher insbesondere eine nicht-sortenspezifische Zelle einer Pflanze, die transient oder stabil mit einer erfindungsgemäßen Nucleotidsequenz transformiert wurde, insbesondere die diese Nucleotidsequenz, beispielsweise in Form eines Antisense-Konstrukts, in ihrem Genom aufweist. Unter einer Pflanze wird ein photosynthetisch aktiver Organismus verstanden, einschließlich Algen, Moose, Farne und höheren Pflanzen.

Die Erfindung betrifft zudem Zellverbände, Gewebe, Organe, Organteile, Zellkulturen, Kalli, differenzierte oder undifferenzierte Zellaggregate, Keimlinge, Protoplasten etc. eines Organismus, die stabil in das Genom integriert oder transient vorhanden mindestens eine mit den erfindungsgemäßen Nucleotidsequenzen transformierte Zelle aufweisen. In besonders bevorzugter Weise betrifft die Erfindung Blätter, Stengel, Samen, Wurzeln, Speicherorgane, Blütenblätter, Blütenorgane etc. einer Pflanze, wobei die genannten Organe mindestens eine mit den erfindungsgemäßen Nucleotidsequenzen stabil oder transient transformierte Zelle aufweisen. In besonders bevorzugter Weise sind die Pflanzen beziehungsweise deren Teile derart transformiert, daß die transformierte Nucleotidsequenz stabil von Generation zu Generation weitervererbt wird.

Die vorliegende Erfindung betrifft neben Zellen, Zellverbänden, Kalli und Pflanzenorganen selbstverständlich auch Pflanzen, insbesondere intakte fertile Pflanzen, die mittels der erfindungsgemäßen Nucleotidsequenz transformiert wurden und in mindestens einer ihrer Zellen mindestens eine dieser Sequenzen aufweisen, insbesondere stabil integriert in ihrem Genom. Die Transformation geschieht, wie nachstehend ausgeführt, vorzugsweise nicht-biologisch, wobei ein Agrobakterium vermittelter Gentransfer als nicht-biologisch verstanden wird. Die erhaltenen Zellen sowie diese Zellen aufweisenden Pflanzengewebe, Pflanzenorgane, Pflanzenteile oder Pflanzen sind nicht sortenspezifisch. Vielmehr ist die Erfindung bei nahezu sämtlichen Pflanzen, Pflanzenfamilien oder Pflanzengattungen anwendbar.

In besonders bevorzugter Weise ist die transformierte Nucleotidsequenz heterolog zu der transformierten Zelle, das heißt natürlicherweise in der transformierten Zelle nicht vorhanden, nicht in der artefiziell erzeugten hohen Kopienzahl vorhanden oder dort nicht, oder nicht in der Zeit exprimiert, in der sie erfindungsgemäß exprimiert wird. In Fällen, in denen die zu transformierende Zelle bereits eine endogene identische oder ähnliche Nucleotidsequenz aufweist, unterscheidet sich die durch die erfindungsgemäße Transformation erhaltene Zelle beispielsweise von der Ausgangszelle dadurch, daß die eingeführte Nucleotidsequenz in einem anderen genetischen Zusammenhang im Genom vorhanden ist, andere regulatorische Elemente aufweist, in Antisenseorientierung zu ihren regulatorischen Elementen angeordnet ist und/oder in erhöhter Kopienzahl vorkommt.

Die Erfindung betrifft demgemäß auch Verfahren zur Herstellung transgener Zellen, wobei die zu transformierende Nucleotidsequenz der Erfindung mittels eines üblichen Transformationsverfahrens, beispielsweise Mikroprojektilbombardement, Agrobakterium-vermittelten Gentransfer, Elektroporation, PEG-vermittelter Transformation oder ähnlichem in die zu transformierende Zelle eingeschleust wird.

Die Erfindung betrifft auch Verfahren zur Herstellung transgener, die erfindungsgemäßen Nucleotidsequenzen aufweisenden Pflanzen, wobei mit den erfindungsgemäßen Nucleotidsequenzen transformierte Zellen oder Zellverbände kultiviert und zu intakten, vorzugsweise fertilen Pflanzen regeneriert werden. Die Kultivierung und Regeneration erfolgt mittels üblicher Verfahren.

Die Erfindung betrifft auch ein Protein mit der Aktivität einer Glutaminsynthetase, insbesondere der GS-1 aus Zuckerrübe, wobei diese durch die erfindungsgemäßen Nucleotidsequenzen, insbesondere die in SEQ ID Nr. 1 gezeigte Nucleotidsequenz codiert wird, besonders bevorzugt eine Aminosäuresequenz, die in SEQ ID Nr. 2 gezeigt wird. Die Erfindung betrifft auch Proteine mit der Aktivität einer Glutaminsynthetase, insbesondere der GS-1 aus Zukkerrübe, wobei diese Sequenz Modifikationen, wie Aminosäureaustausche, Deletionen, Additionen, Inversionen oder ähnliches aufweisen, und wobei das Protein die Aktivität einer GS-1 aus Zuckerrübe aufweist. Die Erfindung betrifft auch Proteine, die auf Aminosäureebene einen Homologiegrad (identische Aminosäuren) von mindestens 90 %, bevorzugt 95 % zu der in der SEQ ID Nr. 2 gezeigten Sequenz aufweisen. Derartige Proteine lassen sich bereitstellen, indem die erfindungsgemäßen Nucleotidsequenzen als Clonierungssonden beziehungsweise Hybridisierungsproben zur Identifizierung und Clonierung homologer, diese Proteine codierender Gene eingesetzt werden.

Die Erfindung betrifft in einer weiteren Ausführungsform mono- oder polyclonale Antikörper gegen eines der vorgenannten Proteine, wobei diese Antikörper ein oder mehrere Epitope der genannten Proteine erkennen und binden.

Die Erfindung betrifft in einer weiteren Ausführungsform Verfahren zur Veränderung des Glutaminstoffwechsels in einer Zuckerrübe, insbesondere zur Modulation der Expression, besonders bevorzugt zur Repression, eines Proteins mit der Aktivität einer Glutaminsynthetase, insbesondere der GS-1 aus Zukkerrübe, wobei der Glutaminsynthetasegehalt der Zuckerrübe verändert wird, indem mindestens eine Zuckerrübenzelle mit einem Vektor gemäß der vorliegenden Erfindung transformiert wird, insbesondere mit einem die erfindungsgemäße Nucleotidsequenz in Antisense-Orientierung aufweisenden Vektor transformiert wird und aus der transformierten Zelle oder einem Zellverbund eine Zuckerrübe regeneriert wird. In vorteilhafter Weise ist eine derartig erzeugte Zuckerrübe dadurch ausgezeichnet, daß das während der Seneszenz in ihren Blättern normalerweise gebildete Glutaminsynthetase GS-1 nicht gebildet wird, da aufgrund des zumindest in den Blättern vorhandenen und dort exprimierten Antisensekonstrukts die Expression der Glutaminsynthetase GS-1 verhindert wird, so daß in den Blättern die Bildung von Glutamin und letztendlich die Einlagerung des Glutamins in die Speicherrübe verhindert wird.

Die Erfindung betrifft jedoch selbstverständlich auch Verfahren zur Veränderung des Glutaminstoffwechsels in Pflanzen, insbesondere Zuckerrüben, gemäß der der Glutaminsynthetasegehalt in bestimmten Zellen oder Organen, gegebenenfalls zu bestimmten Zeiten, erhöht wird, insbesondere, indem Genkonstrukte transformiert werden, die eine konstitutive und/oder verstärkte Expression der erfindungsgemäßen Nucleotidsequenzen ermöglichen.

Weitere vorteilhafte Ausgestaltungen der Erfindung ergeben sich aus den Unteransprüchen.

Die Erfindung wird anhand von Beispielen und dazugehöriger Zeichnungen näher erläutert.

SEQ ID Nr. 1 zeigt den translatierten Bereich der cDNA-Sequenz der GS-1 aus Zuckerrübe.

SEQ ID Nr. 2 zeigt die Aminosäuresequenz der GS-1 aus Zuckerrübe.

SEQ ID Nr. 3 zeigt die Gesamt-cDNA Sequenz der GS-1 aus Zuckerrübe.

Die Figuren zeigen:

Figur 1 einen Western-Blot erfindungsgemäß erhaltener GS-1,

Figur 2 ein Autoradiogramm des Western-Blots nach Figur 1 und

Figur 3 eine schematische Darstellung der in den Figuren 1 und 2 erhaltenen Ergebnisse.

Beispiel 1: Clonierung der cDNA für die GS-1

Gesamt-RNA wurde aus seneszenten Zuckerrübenblättern extrahiert. Dazu wurden 20 g Blattmaterial aus seneszenten Zuckerrübenblättern in flüssigem Stickstoff gemörsert und in 100 ml Aufnahmepuffer (50 mM Tris-HCl pH 9,0, 100 mM NaCl, 10 mM EDTA, 2% w/v SDS und 0,2 mg/ml Proteinase K) überführt. Dieser Ansatz wurde zweimal mit Phenol/Chlorophorm/Isoamylalkohol (25/24/1) phenolisiert, gefällt (1/10 Volumen 3M NaAc, pH 6,5, ein Volumen Isopropanol, 2 Stunden bei -20°C) und mit 70% Ethanol gewaschen. Nach Aufnahme in 10 ml $H_2O$, 10 µg/ml Proteinase K und 2 x Fällen mit $\frac{1}{4}$ Volumen 10 M LiCl für 16 Stunden bei 0°C wurde die Gesamt-RNA in 2 ml $H_2O$ mit 10 µg/ml Proteinase K aufgenommen. Es wurden 5 mg Gesamt-RNA erhalten.

Anschließend wurde über eine Oligo-dT-Cellulosesäule Poly $(A)^+$ m-RNA isoliert. Dazu wurden 2 ml Gesamt-RNA mit 25 ml Bindungspuffer (400 mM NaCl, 10 mM Tris-HCl, pH 7,5 und 2% SDS) und Oligo-dT-Cellulose (200 mg Oligo-dT-Cellulose) für 30 Minuten bei Raumtemperatur und unter sanftem Schütteln inkubiert. Das Gemisch wurde in eine Glassäule mit Baumwollfritte überführt und mit einem Waschpuffer (100 mM NaCl, 10 mM Tris-HCL, pH 7,5, 0,2% SDS) tropfenweise bis zur Konstanz der $OD_{260}$ bei 0,005 gespült. Anschließend wurde mit 10 ml Elutionspuffer (10 mM Tris-HCL, pH 7,5) bei 55°C eluiert. Daraufhin wurde mit 1/10 Volumen 3 M NaAc, pH 6,5 und 2 Volumen Ethanol bei -20°C für zwei Stunden gefällt und der Ansatz in 10 ml Bindungspuffer aufgenommen. Daraufhin wurde die Säulenaufreinigung wiederholt, das Eluat vor dem Fällen phenolisiert und das mRNA-Pellet in TE-Puffer aufgenommen. Es wurden 50 µg Poly $(A)^+$ m-RNA erhalten.

cDNA wurde mittels eines cDNA-Synthesis-Kits von Boehringer Mannheim nach einem Standardprotokoll (5 µg mRNA eingesetzt) hergestellt. Die erhaltene cDNA wurde in Lambdavektoren (NM 1149) ligiert. Dazu wurden 4 µg NM 1149 (EcoR I verdaut) und 0,2 µg cDNA mit EcoR I-Linkern eingesetzt. Nach der Ligation wurde die DNA in Phagen (NM 1149) verpackt. Dazu wurde der Gigapack@ II Gold Packaging Extract der Firma Stratagene nach dem Standardprotokoll eingesetzt, wobei 4 µg DNA verwendet wurden und ein Titer von 140000 pfu erhalten wurde.

Die erhaltene cDNA-Bank wurde ebenso wie eine cDNA-Bank aus Wurzelgewebe von Zuckerrüben (Lambda ZAP® II Library, Stratagene, cDNA über EcoR I und Not I in das Lambda ZAP® II Vektorsystem inseriert, Titer: 250000 pfu, Vektor pBluescript® SK (-) mit Insertion nach Standardprotokoll durch in vivo Excision aus Lambda ZAP® II isoliert) mit einer heterologen Sonde aus Tabak gescreent. Die heterologe Tabaksonde ist in Becker at al (1992) Plant. Molec. Biol. 19, 367-379 beschrieben. Für das Screening wurden E.coli Bakterien mit den Lambda-Phagen infiziert und ausplattiert. Anschließend wurde die Phagen-DNA aus lysierten Bakterien auf NC-Membranen (Plaquelift) übertragen und die membrangebundene DNA mit einer radioaktiv markierten GS-1 cDNA-Sonde aus Tabak hybridisiert.

Das Screening der cDNA-Bank mit der heterologen Sonde aus Tabak wurde wie folgt durchgeführt. Zunächst wurde eine Vorhybridisierung mit 6 x SSPE, 0,1% SDS, 5 x Denhardt's Reagenz und 500 µg/ml denaturiertem Heringssperma bei 61°C für zwei Stunden durchgeführt. Anschließend wurde die Hybridisierung bei 61°C für 16 Stunden mit einer Lösung aus 3 x SSPE, 0,1% SDS, 5 x Denhardt's Reagenz und 500 µg/ml denaturiertem Heringssperma durchgeführt. Das Waschen wurde mit 2 x SSC und 0,1% SDS bei 61°C für 2 x 15 Minuten durgeführt. Anschließend wurde ein Waschschritt mit 1 x SSC und 0,1% SDS bei 61°C für 15 Minuten durchgeführt.

Es wurde ein Autoradiogramm der Membranfilter entwickelt, positive Phagen isoliert und die entsprechende DNA extrahiert. Die aufgefundene cDNA wurde in das Plasmid pBluescript SK (Stratagene) subcloniert und sequenziert. Die Nucleotidsequenz des translatierten Bereichs der cDNA inclusive des Translationsstartcodons ATG ist in SEQ ID Nr. 1 dargestellt und weist eine Länge 1.068 bp auf. Die Gesamtsequenz der cDNA ist in SEQ ID Nr. 3 dargestellt und weist eine Länge von 1543 bp auf. Das Startcodon befindet sich in Position 199 bis 201. Der translatierte Bereich endet bei Position 1266. Ein Polyadenylierungssignal liegt im Bereich der Nucleotide 1478 bis 1508.

Die von der SEQ ID Nr. 1 abgeleitete Aminosäuresequenz weist eine Länge von 356 Aminosäuren auf und ist in der SEQ ID Nr. 2 dargestellt. Die Aminosäuresequenz stellt die Aminosäuresequenz der Untereinheit P der GS-1 aus Zuckerrübe dar. Das Protein ist ca. 42 kDa groß und stellt die einzige Untereinheit der GS-1 Isoform dar, die in Form eines Homooktamers in seneszenten Blättern der Zuckerrübe vorliegt.

Beispiel 2: In vitro Transcription und Translation der P-Untereinheit

Die in SEQ ID Nr. 1 dargestellte Nucleotidsequenz wurde in vitro transcribiert und translatiert. Die dabei eingesetzte clonierte GS-1-DNA-Sequenz entstammte der in Beispiel 1 erwähnten Zuckerrüben cDNA aus Wurzelgewebe. Um zu ermitteln, für welche GS-1-Untereinheit diese DNA-Sequenz codiert, wurde eine in vitro Transcription und Translation mit dem "Linked in vitro SP 6/T7 Transcription/Translation Kit-radioaktiv" Kit von Boehringer Mannheim durchgeführt. Dazu wurden 0,5 µl (0,5 µg) Plasmid-DNA (pBluescript® SK (-) mit dem GS-1 Insert), 5 µl T7 Transcriptionspuffer und 14,5 µl $H_2O$ für 15 Minuten bei 30°C inkubiert. Anschließend wurden 10 µl Transcriptions-Reaktionslösung, 1,6 µl $^{35}$S-Methionin und 38,4 µl Translations-Mix für 1 Stunde bei 30°C inkubiert.

Außerdem wurde aus 5 g Zuckerrübenblättern unterschiedlicher Altersstadien (um alle GS-1-Untereinheiten zur eindeutigen Identifizierung zu erhalten) ein Proteinextrakt hergestellt. Dieser Extrakt wurde über eine FPLC gereinigt und die Fraktionen mit den höchsten GS-1-Aktivitäten eingeengt. Die Proteinbestimmung erfolgte nach Bradford und ergab eine Proteinkonzentration von ca. 1 µg/µl. Sowohl dieser Extrakt als auch das Reaktionsgemisch der in vitro Translation (mit dem radioaktiv markiertem GS-1 Protein) wurden mit dem gleichen Volumen Harnstoffbeladungspuffer versetzt. Davon wurden je 20 µl zusammen als Probe für eine isoelektrische Fokussierung (IEF) auf ein Acrylamid-Kapillargel gegeben (1. Dimension). Eine isoelektrische Fokussierung erfolgte bei 190 V für 16 h.

Zusammen mit einem Protein-Größen-Standard wurde das Kapillargel auf ein SDS Gel überführt, um die Proteine ihrer Größe nach aufzutrennen (2. Dimension). Eine SDS-PAGE erfolgte bei 140 V für 2 h. Von diesem Gel wurde ein Western-Blot angefertigt (500 mA; 1 h).

Die Nitrocellulosemembran wurde mit Ponceau Rot angefärbt und die Banden des Größenstandards mit Bleistift markiert.

Nach einer Blocking-Behandlung (1 h) wurde die Membran mit dem 1. Antikörper (anti-GS; Antikörper gegen Gersten-GS, Roger Wallsgrove, Rothamsted Experimental Station, Harpenden, UK), 1:3000 in Blockinglösung) inkubiert (16 h; RT). Die Membran wurde dann 3 x mit TBS gewaschen und mit dem 2. Antikörper (1:2000 in Blockinglösung) inkubiert (3 h; RT). Nach erneut dreimaligem Waschen erfolgte die Farbreaktion mit NBT und BCIP durch die Alkalische Phosphatase (10-20 min; 37°C; dunkel). Die getrocknete Membran mit den farbig markierten Spots für die GS-1 Untereinheiten wurde mit einem Röntgenfilm belegt (Exposition: 16 h; RT; dunkel).

Figur 1 zeigt den mit GS-Antikörpern behandelten und gefärbten Membranfilter (Western Blot). Links ist der Proteingrößen-Standard aufgetragen. Der pH-Gradient verläuft hier von links pH 6 nach rechts pH 4. Auf Höhe der 43 kDa Bande erkennt man die 4 Spots der GS-1 Untereinheiten (vergleiche Figur 3, hier allerdings seitenverkehrt). Der Spot für die Untereinheit P ist markiert (Pfeil).

Figur 2 zeigt das Autoradiogramm der Membran, auf dem links die Banden des Größenstandards eingezeichnet sind und auf dem rechts in Höhe der 43 kDa Bande ein Spot auftritt. Dieser Spot wurde durch das in vitro transcribierte und translatierte radioaktiv markierte Protein erzeugt. Bringt man das Autoradiogramm mit der Membran in Deckung, läßt sich dem einzelnen Spot des Films ein Spot der Membran zuordnen. Dieser Spot wurde als Untereinheit "P" identifiziert (vergleiche Figur 3).

Figur 3 stellt ein Schema der durch GS-Antikörper markierbaren Proteine dar. Unten wird die Richtung des pH-Gradienten angegeben (1. Dimension) von links pH 4 bis rechts pH 6. Rechts sind die Banden des Größenstandards (2. Dimension) von oben 43 kDa bis unten 30 kDa dargestellt. Die hier weißen Punkte a, b, c und d zeigen die Positionen der GS-2 Untereinheiten an, welche ebenfalls durch den Antikörper erkannt werden, aber durch die FPLC von der GS-1 getrennt werden können. Auf Höhe der 43 kDa Bande sind vier schwarze Punkte s, i, p und w, die aufgrund ihrer Größe als diejenigen Untereinheiten identifiziert werden konnten, welche das Oktamer der GS-1 bilden. Die anderen schwarzen Punkte u, v, $x_1$, $x_2$, y und z stellen möglicherweise Abbauprodukte der GS-Proteine dar.

Beispiel 3: Herstellung von transgenen Zuckerrüben

Es wurde eine Reihe von Konstrukten hergestellt, die jeweils einen in Pflanzen exprimierbaren Promotor, nämlich den CaMV 35 S-Promotor, jeweils einen Abschnitt des erfindungsgemäßen Gens der GS-1-Untereinheit aus der Zuckerrübe in antisense-Orientierung sowie jeweils den NOS-Terminator enthielten. Die eingesetzten Abschnitte des erfindungsgemäßen Gens unterschieden sich. voneinander. Die so hergestellten Gen-Kassetten wurden in den Binärvektor (BIN19 mit Kanamycinresistenz) ligiert und Agrobacterium tumefaciens (mit Rifampicinresistenz) mit den erstellten Binärvektoren mittels Elektroporation transformiert. Die Transformanten wurden auf Rifampicin und Kanamycin selektiert. Anschließend wurden Zuckerrübenblattscheiben beziehungsweise Blattstiele in einer Suspension mit transformierten Agrobakterien transformiert und Kallus- und Sproßbildung mit den Pflanzenhormonen NAA und BAP induziert. Nach Selektion auf kanamycinhaltigem Medium und Regeneration zu intakten Pflanzen gemäß üblicher Protokolle konnte mittels Messungen der GS1-Enzymaktivitäten, SDS-Gelelektrophoresen, 2D-PAGE und Northern Blot-Analysen nachgewiesen werden, daß sämtliche eingesetzten Konstrukte mit den unterschiedlichen Genabschnitten in den Blättern der transgenen, regenerierten Pflanze vorhanden und aktiv waren sowie zur Unterdrückung der Glutaminsynthetase-Aktivität und -Bildung in seneszenten Blättern der Zuckerrübe führten.

## Patentansprüche

1. Nucleotidsequenz für die Modulation der Expression eines Proteins mit der Aktivität einer Glutaminsynthetase ausgewählt aus der Gruppe bestehend aus

   a) der DNA-Sequenz der SEQ ID Nr. 1 und 3,

   b) einer Nucleotidsequenz, die die Aminosäuresequenz der SEQ ID Nr. 2 codiert,

   c) einer zu den Nucleotidsequenzen von a) oder b) komplementären Nucleotidsequenz, und

   d) einer Nucleotidsequenz, die einen Homologiegrad von mindestens 80 % zu den in der SEQ ID Nr. 1 oder 3 gezeigten Sequenzen aufweist,

   wobei die Nucleotidsequenz aus der Zuckerrübe ist.

2. Vektor, enthaltend eine Nucleotidsequenz nach Anspruch 1.

3. Vektor nach Anspruch 2, wobei der Vektor ein Plasmid oder ein viraler Vektor ist.

4. Vektor nach einem der Ansprüche 2 oder 3, wobei der Nucleotidsequenz mindestens eine regulatorische Nucleotidsequenz zugeordnet ist.

5. Vektor nach einem der Ansprüche 2 bis 4, wobei 5'-wärts von der Nucleotidsequenz ein die Expression der Nucleotidsequenz steuernder Promoter angeordnet ist.

6. Vektor nach einem der Ansprüche 2 bis 5, wobei 3'-wärts von der Nucleotidsequenz ein 3'-Polyadenylierungssignal angeordnet ist.

7. Vektor nach einem der Ansprüche 2 bis 6, wobei die regulatorische Sequenz induzierbar ist.

8. Vektor nach einem der Ansprüche 2 bis 7, wobei die regulatorische Sequenz der Expression der Nucleotidsequenz Gewebe- und/oder Zeitspezifität verleiht.

**9.** Vektor nach einem der Ansprüche 2 bis 8, wobei die Nucleotidsequenz Antisenseorientierung zum Promoter aufweist.

**10.** Zelle, enthaltend einen Vektor nach einem der Ansprüche 2 bis 9.

**11.** Zelle nach Anspruch 10, die eine Bakterienzelle, eine Hefezelle oder eine Pflanzenzelle, insbesondere eine Zuckerrübenzelle, ist.

**12.** Pflanze, enthaltend mindestens eine Zelle nach einem der Ansprüche 10 oder 11.

**13.** Samen einer Pflanze nach Anspruch 12, wobei dieser Samen eine Nucleotidsequenz nach Anspruch 1 enthält.

**14.** Verfahren zur Veränderung des Glutaminstoffwechsels in einer Zuckerrübe, wobei die Synthese der Glutaminsynthetase in der Zuckerrübe verhindert oder reduziert wird, indem mindestens eine Pflanzenzelle mit einem Vektor gemäß Anspruch 9 transformiert und die Zuckerrübe regeneriert wird.

**15.** Verfahren zur Herstellung einer transgenen Zuckerrübe, die einen veränderten Glutaminstoffwechsel aufweist, und wobei dieser auf einer Verringerung des Gehalts an Glutaminsynthetase in der Pflanzenzelle beruht, wobei mindestens eine Pflanzenzelle mit einem Vektor gemäß Anspruch 9 transformiert und die Pflanzenzelle zu einer intakten Pflanze regeneriert wird.

## Claims

**1.** Nucleotide sequence for modulating the expression of a protein having the activity of a glutamine synthetase selected from the group consisting of

a) the DNA sequence of SEQ ID No. 1 and 3,

b) a nucleotide sequence which encodes the amino acid sequence of SEQ ID No. 2,

c) a nucleotide sequence which is complementary to the nucleotide sequence of a) or b), and

d) a nucleotide sequence which has a degree of homology of at least 80 % to the sequences shown in SEQ ID No. 1 or 3,

wherein the nucleotide sequence is from sugarbeet.

**2.** Vector comprising a nucleotide sequence as claimed in claim 1.

**3.** Vector according to claim 2, wherein the vector is a plasmid or a viral vector.

**4.** Vector according to one of the claims 2 or 3, wherein at least one regulatory nucleotide sequence is assigned to the nucleotide sequence.

**5.** Vector according to one of the claims 2 to 4, wherein a promoter controlling the expression of the nucleotide sequence is arranged 5'-wards of the nucleotide sequence.

**6.** Vector according to one of the claims 2 to 5, wherein a 3'-polyadenylation signal is arranged 3'-wards of the nucleotide sequence.

**7.** Vector according to one of the claims 2 to 6, wherein the regulatory sequence is inducible.

**8.** Vector according to one of the claims 2 to 7, wherein the regulatory sequence confers tissue specificity and/or time specificity to the expression of the nucleotide sequence.

**9.** Vector according to one of the claims 2 to 8, wherein the nucleotide sequence has antisense orientation to the promoter.

**10.** Cell comprising the vector according to one of the claims 2 to 9.

**11.** Cell according to claim 10, which is a bacterial cell, a yeast cell or a plant cell, in particular a sugarbeet cell.

**12.** Plant comprising at least one cell according to one of the claims 10 or 11.

**13.** Seed of a plant according to claim 12, wherein this seed comprises a nucleotide sequence according to claim 1.

**14.** Method for altering glutamine metabolism in a sugarbeet, wherein synthesis of glutamine synthetase in the sugarbeet is prevented or reduced by transforming at least one plant cell with the vector according to claim 9, and regenerating the sugarbeet.

**15.** Method for producing a transgenic sugarbeet, which shows altered glutamine metabolism, wherein the latter is based on a reduction of the content of glutamine synthetase in the plant cell and wherein at least one plant cell is transformed with the vector according to claim 9, and the plant cell is regenerated to an intact plant.

**Revendications**

**1.** Séquence nucléotidique pour la modulation de l'expression d'une protéine avec l'activité d'une glutamine synthétase sélectionnée dans le groupe constitué par :

a) la séquence d'ADN de SEQ ID No 1 et 3 ;

b) une séquence nucléotidique qui code pour la séquence d'acides aminés de SEQ ID No 2 ;

c) une séquence nucléotidique complémentaire aux séquences nucléotidiques de a) ou b) ; et

d) une séquence nucléotidique qui présente un taux d'homologie d'au moins 80 % par rapport aux séquences présentées dans SEQ ID No 1 ou 3,

dans laquelle la séquence nucléotidique provient de la betterave à sucre.

**2.** Vecteur contenant une séquence nucléotidique d'après la revendication 1.

**3.** Vecteur selon la revendication 2, dans lequel le vecteur est un plasmide ou un vecteur viral.

**4.** Vecteur selon l'une ou l'autre des revendications 2 ou 3, dans lequel au moins une séquence nucléotidique régulatrice est associée à la séquence nucléotidique.

**5.** Vecteur selon l'une quelconque des revendications 2 à 4, dans lequel un promoteur contrôlant l'expression de la séquence nucléotidique est agencé à l'extrémité 5' de la séquence nucléotidique.

**6.** Vecteur selon l'une quelconque des revendications 2 à 5, dans lequel un signal de 3'-polyadénylisation est agencé à l'extrémité 3' de la séquence nucléotidique.

**7.** Vecteur selon l'une quelconque des revendications 2 à 6, dans lequel la séquence régulatrice peut être induite.

**8.** Vecteur selon l'une quelconque des revendications 2 à 7, dans lequel la séquence régulatrice de l'expression confère à la séquence nucléotidique la spécificité tissulaire et/ou temporelle.

**9.** Vecteur selon l'une quelconque des revendications 2 à 8, dans lequel la séquence nucléotidique présente une orientation antisens par rapport au promoteur.

**10.** Cellule contenant un vecteur selon l'une quelconque des revendications 2 à 9.

**11.** Cellule selon la revendication 10, qui est une cellule de bactérie, une cellule de levure et/ou une cellule végétale, notamment une cellule de betterave à sucre.

**12.** Plante contenant au moins une cellule selon l'une quelconque des revendications 10 ou 11.

**13.** Graine d'une plante selon la revendication 12, dans laquelle cette graine contient une séquence nucléotidique selon la revendication 1.

**14.** Procédé pour modifier le métabolisme de la glutamine dans une betterave à sucre, dans lequel la synthèse de la glutamine synthétase est empêchée dans la betterave à sucre ou y est réduite, une cellule végétale au moins étant transformée à l'aide d'un vecteur selon la revendication 9 et la betterave à sucre étant régénérée.

**15.** Procédé pour la fabrication d'une betterave à sucre transgénique, qui présente un métabolisme de la glutamine modifiée, et dans lequel ce dernier s'appuie sur une réduction de la teneur en glutamine synthétase dans la cellule végétale, une cellule végétale au moins étant transformée à l'aide d'un vecteur selon la revendication 9 et la cellule végétale étant régénérée en une plante intacte.

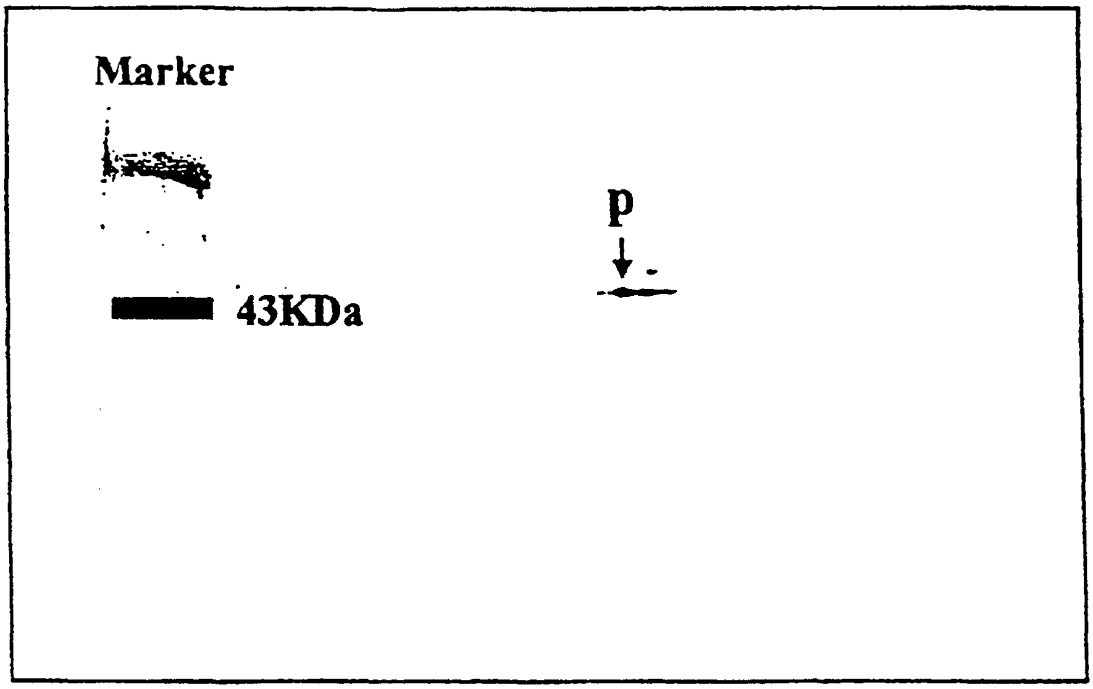


Figur 1

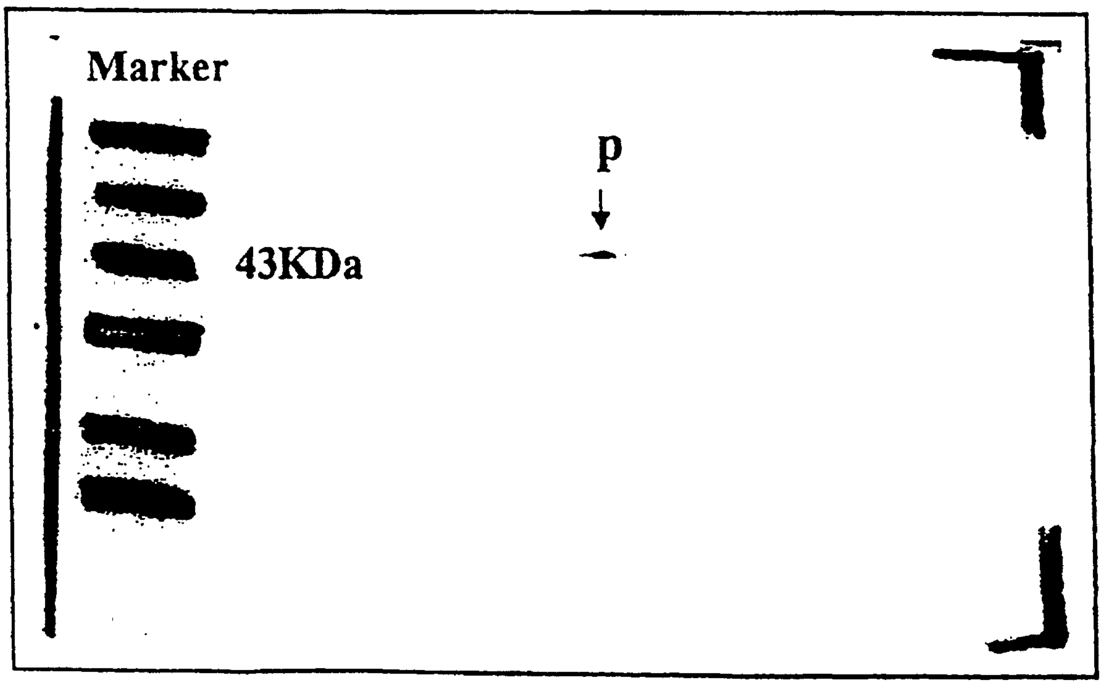


Figur 2

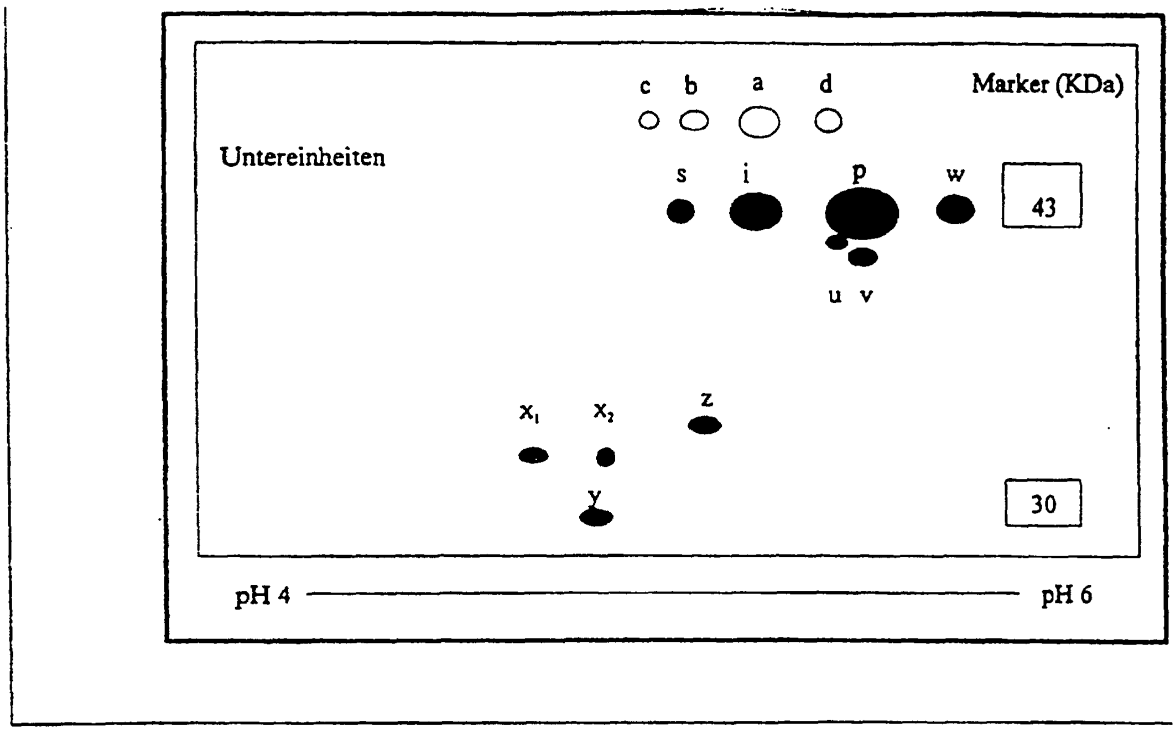


Figur 3